(19) Europäisches Patentamt
European Patent Office
Office européen des brevets

(11) Publication number: **0 111 413**
**B1**

(12) **EUROPEAN PATENT SPECIFICATION**

(45) Date of publication of patent specification: **06.08.86**

(51) Int. Cl.$^4$: **C 07 C 147/02,**
A 61 K 31/275, A 01 N 37/34

(21) Application number: **83307380.2**

(22) Date of filing: **05.12.83**

(54) **Novel sulfone, process for its preparation, composition containing it and use thereof.**

(30) Priority: **06.12.82 US 446898**

(43) Date of publication of application:
**20.06.84 Bulletin 84/25**

(45) Publication of the grant of the patent:
**06.08.86 Bulletin 86/32**

(84) Designated Contracting States:
**BE CH DE FR GB IT LI NL SE**

(56) References cited:
**US-A-3 441 614**
**US-A-4 079 148**

(73) Proprietor: **STAUFFER CHEMICAL COMPANY**
**Westport Connecticut 06881 (US)**

(72) Inventor: **Mantis, Andrew P.**
**S. Hillside Lake Road**
**Wappinger Falls, NY 12590 (US)**
Inventor: **Simmons, Kirk A.**
**126 Brook Street**
**Scarsdale NY 10583 (US)**

(74) Representative: **Smith, Sydney et al**
**Elkington and Fife High Holborn House 52/54**
**High Holborn**
**London WC1V 6SH (GB)**

**EP 0 111 413 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to the use of a known sulphone, as a microbiocide and as a post-emergent herbicide. A particular use of the sulphone is as a fungicide in paints.

Many sulfones are known to possess agricultural or biocidal activity. For example, some sulfones are taught to be useful for the control of microorganisms and specific materials have been shown to possess bactericidal, fungicidal or algicidal activity. Similarly, some sulfones have been taught to be useful as insecticides or herbicides.

One patent, U.S. Patent 3,140,307, relates to certain toxic sulfones which are arylsulfonyl haloalkanenitriles. Certain of these materials are shown to have some bactericidal, insecticidal or, biocidal activity.

Another patent, U.S. Patent 3,441,614, relates to a process for the production of certain sulfones. This patent pertains to the production of beta-chloroorganosulfones, employed, as a catalyst, an iron compound or a copper compound, which may be used with a source of chloride ion, such as triethyl ammonium chloride, and indicates that beta-chlorosulfones are of interest as pesticides and as intermediates for the manufacture of certain alpha, beta-unsaturated sulfones, said to be known as bactericidic and fungicidal compounds. This patent describes the preparation of 3 - (methylsulfonyl) - 2 - chloropropanenitrile which is represented by the formula:

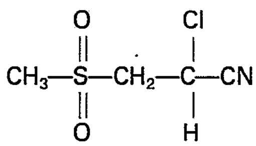

Another patent, U.S. Patent No. 4,079,148, describes certain antimicrobial sulfones. While this patent relates to a relatively broad class of sulfones, it is directed to and claims certain unsaturated monohalogenated sulfones and certain saturated, dihalogenated sulfones, which are said to have a variety of microbiocidal activities.

We have now found that the said sulfone, 3 - (methylsulfonyl) - 2 - chloropropanenitrile has outstanding microbial activity as well as post-emergent herbicidal activity.

In one aspect of the invention there is provided an antimicrobial composition containing an effective amount of the said sulfone, optionally with an adjuvant. In another aspect of the invention there is provided a herbicidal composition containing a herbicidally effective amount of the said sulfone optionally with an adjuvant.

The said sulfone may be prepared as described in U.S. Patent 3,441,614, by the reaction of methyl sulfonyl chloride with acrylonitrile in the presence of a catalyst, in accordance with the following schematic equation:

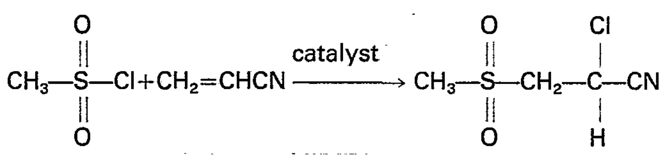


Thus, for example, one mole of methylsulfonyl chloride may be reacted with from 1 to 6 moles, and preferably an excess, for example, from 2 to 4 moles, of acrylonitrile in the presence of a catalyst.

The catalyst may be a copper catalyst, such as CuCl or $CuCl_2 \cdot 2H_2O$, and is preferably employed in an amount of from 1 to 5 percent by weight of the acrylonitrile. Preferably, an amine hydrochloride, for example triethyl amine hydrochloride, is also employed in an amount of from 100 to 500 weight percent of the copper catalyst.

The reaction may be conducted at a temperature of from 30 to 100°C for a period of 1 to 36 hours and is preferably conducted at the reflux temperature of acrylonitrile, e.g. from 75 to 85°C, for a period of from 16 to 24 hours.

Normally, the reaction will be conducted at atmospheric pressure, but it may be conducted at a pressure of from about 14 to 28 p.s.i.a. (1—2 Bar). The reaction is preferably conducted in an inert atmosphere, such as an atmosphere of nitrogen gas.

After the reactants have been heated for the indicated time, as set forth above, the reaction mixture is brought to ambient temperature, for example, by being allowed to stand at room temperature for a period of 1 to 4 hours. The resulting reaction product is a solid which can be triturated with a solvent, for example, a methylene dichloride-hexane mixture, air dried and recovered.

The sulfone used in the compositions of the present invention has been found to be useful as a fungicide and bactericide against organisms found to cause problems in paint compositions.

In order to test the effectiveness of this compound, against organisms which adversely affect paints, the following tests were conducted.

Test procedure

A Minimum Inhibitory Concentration test with bacteria and fungus was performed in nutrient broth. The test compound was added to sterile broth at 0.1% (1000 PPM) and diluted sequentially with sterile broth to yield required levels. One drop of an actively-growing 24-hour culture was added to each level and incubated for a period of 24 hours at a temperature of 30°C. At the end of the time period, an aliquot from each tube was placed on nutrient agar and observed for growth.

The results of the tests are shown in Table I, below, wherein "Compound 1" is 3 - (methylsulfonyl) - 2 - chloropropanenitrile.

TABLE I

Bacterial and fungal minimum inhibitor concentration of compound 1

| | Bacterial—Pseudomonas aeruginosa | | | | |
|---|---|---|---|---|---|
| | 1000 PPM | 500 PPM | 250 PPM | 125 PPM | 62.5 PPM |
| Compound 1—pH 4 | – | – | – | – | – |
| Compound 1 buffered to pH 6.8 | – | – | – | – | – |
| | Fungal—Alternaria solani | | | | |
| | 1000 PPM | 500 PPM | 250 PPM | 125 PPM | 62.5 PPM |
| Compound 1—pH 4 | – | – | – | – | + |
| Compound 1 buffered to pH 6.8 | – | – | – | + | + |

Legend: + = Growth
– = No Growth

As can be seen from Table I, activity against bacteria and against fungus was shown at low concentrations and at two pH levels. Although it is believed that this compound will be effective in paint compositions, its activity in paint compositions has not yet been successfully demonstrated.

The present compound has also been found to exhibit activity as a post emergence herbicide.

The post emergence herbicidal activity of the present compound was evaluated as follows:

Test procedure

Aluminum planting flats measuring 15.2×22.9×8.9 cm were filled to a depth of 7.6 cm with loamy sand soil, containing 50 parts per million (ppm) each of the commercial fungicide cis - N(trichloromethyl)thio - 4 - cyclohexane - 1,2 - dicarboximide (Captan®) and 17-17-17 fertilizer (percentages of $N—P_2O_5—K_2O$) on a weight basis). Several rows were impressed across the width of each flat and a variety of seeds of both grass and broadleaf weed species were planted, one species per row. The weed species used are listed below:

Broadleaf weeds:

| | |
|---|---|
| A. Annual morning glory | Ipomoea purpurea |
| B. Cocklebur | Xanthium sp. |
| C. Jimsonweed | Datura stramonium |
| D. Velvetleaf | Abutilon theophrasti |
| E. Mustard | Brassica sp. |
| F. Nightshade | Solanum sp. |
| G. Pigweed | Amaranthus sp. |

Grasses:

| | |
|---|---|
| H. Yellow nutsedge | Cyperus esculentus |
| I. Downybrome | Bromus Tectorum |
| J. Foxtail | Setaria sp. |
| K. Annual ryegrass | Lolium multiflorum |
| L. Watergrass | Echinochloa crusgalli |
| M. Shattercane | Sorghum bicolor |
| N. Wild oat | Avena fatua |

The broad leaf species were seeded first, and the grasses were seeded four days later. Ample seeds of

each species were planted to produce 20 to 50 seedlings per row after emergence, depending on the size of each plant.

Ten days after the grasses were seeded, the emerged seedlings of all species were sprayed with aqueous solutions of the test compounds. The solutions were prepared to such dilutions that a spray rate of 80 gallons per acre (750 liters per hectare) gave from 0.5 to 4.0 pounds of test compound per acre (0.56 to 4.48 kilograms per hectare) as desired for each test. Additional flats not treated at all were used as standards for measuring the extent of weed control in the treated flats.

Nineteen days later, the test flats were compared to the standards and the weeds in each row were rated visually in terms of percent control ranging from 0% to 100%, with 0% representing the same degree of growth as the same row in the standard and 100% representing complete kill of all weeds in the row. All types of plant injury were taken into consideration. The results are shown in Table II, wherein "Compound 1" is 3 - (methylsulfonyl) - 2 - chloropropanenitrile.

TABLE II
Herbicidal test results

| | | Percent control | | | | | | | | | | |
|---|---|---|---|---|---|---|---|---|---|---|---|---|
| | | Broadleaf weeds—grasses | | | | | | | | | | |
| Test compound | Application rate (lb/A) | A | B | C | D | E | — | J | K | L | M | N |
| Compound 1 | 4.0 | 75 | | | 75 | 40 | | 75 | | 65 | | 30 |

The present compound has also been found to exhibit activity as an insecticide.

In addition to the above-described use for the compound of the present invention, it is believed that the present compound is generally useful as a fungicide, not only in paints, but also as a fungicide for other uses, including the protection of plastics and polymer materials and as an agricultural fungistat.

Similarly, the present compound is believed useful, generally, as a miticide, an algicide and as a microbiological toxicant for the control of bacteria, molds, actinomycetes and the like.

It is believed that the present compound is also useful in the prevention and control of bacterial infection and of decomposition and decay caused by mildew and molds.

The present compound, it is believed, can be employed for the protection of organic materials subjected to deterioration by rotting, such as leather, fur, pulp, paper, textiles, rope, rubber, latex, plastics and paint. Incorporation of the present material, where it functions as a fungistat, in such organic materials is especially desirable when the materials are exposed to conditions favoring microbiological growth.

It is also believed that the present compound can be used to protect wood which is buried in the ground, textiles exposed to damp, for example, lawn furniture, awnings and the like, and in marine paints and lacquers subject to algae and fungal attack.

The present compound is also believed useful as a seed protectant and soil sterilant, and to be active against sulphate-reducing bacteria.

It is believed that the present compound can be added as a biocide to oil field injection flood waters for the prevention of pipe plugging caused by releasing bacteria, and to be useful as a biocide for addition to cooling water systems, as paper mill water systems and the like.

The present compound is also believed to exhibit germicidal activity and to be effective as a disinfectant, useful for disinfecting and sterilizing surgical instruments, dairy equipment, and eating utensils.

The present compound can be applied directly to the material being treated, for example, by incorporation in a soap or a cream. It may also be employed in a dilute concentration for most applications, and is preferably employed in conjunction with a carrier or a diluent. The choice of diluent is determined by the use of the composition as use of concentration of the active ingredient in the diluent. Thus, by a mixture of an inert pulverulent carrier such as talc, bentonite, diatomaceous earth and the like, there can be prepared compositions suitable for a mixture with seeds and the like to afford protection from microbiological attack in the soil.

Solutions of the present compound in inorganic solvents such as kerosene, may be applied as a spray or impregnating bath. Suitable formulations of the present compound for application to articles subject to microbiological attack can also prepared by mixing the compound with an emulsifying agent suitably in the presence of organic solvent and diluting with water to form an aqueous emulsion.

Suitable emulsifying agents include alkyl benzene sulfonates and polyalkylene glycols.

Aqueous emulsions of the present compound may also be used as disinfectant solution, for example, to wash floors and walls and the like.

In another embodiment of the present invention, standard paint formulations may be used as diluent or carrier for the compound of the present invention, which can function in prevention mold growth. The paints can be applied to surfaces which are thereby rendered resistant to the growth of lower organisms.

The present sulfone can be admixed with carriers, which are active of themselves, for example, with hormones, with buffering and/or softening agents and the like.

As indicated above, the present compound may be used as a biocide to prevent, control or inhibit the growth of microorganisms, such as fungi. Thus, another embodiment of this invention comprises a method of controlling microorganisms by applying to the locus where such control is desired.

An effective amount of the present compound varies with the use and the degree of control desired. For the various uses of the compound of this invention to control microorganisms, one skilled in the art will be able, without undo experimentation, determine the effective amount of the compound required to provide the desired degree of control.

Throughout this specification, all parts and percentages are by weight, unless otherwise specified.

Preparation of 3-(methylsulfonyl)-2-chloropropanenitrile

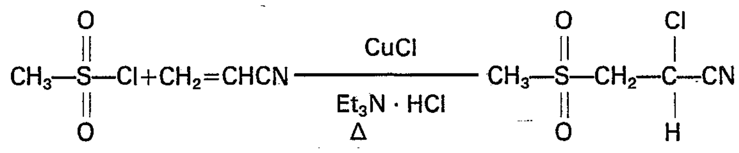


11.5 g (100 m mole) of $CH_3—SO_2—Cl$, 26 ml (21.2 g, 400 m mole) of acrylonitrile, 200 mg (2 m mole) of CuCl and 400 mg (3 m mole) of $Et_3N \cdot HCl$ were mixed and heated to reflux in an atmosphere of nitrogen at about atmospheric pressure. A temperature of 84°C was maintained. Reaction mixture was found to darken. The reaction was continued, under reflux conditions for about 18 hours, after which the reaction mixture was found to be perfectly clear and amber in color. Refluxing was continued for a total time of 26 hours; after which the mixture solidified and was dissolved in $CH_2Cl_2$/ethyl acetate, washed with water, dried and concentrated to a pale yellow oil by vacuum distillation of the solvents. The yield of 3 - (methylsulfonyl) - 2 - chloropropanenitrile was found to be 16.0 g. The product solidified, upon standing, and was found to have a melting point of 60—62°C. It was submitted for NMR analysis and was found to be 95% pure 3 - (methylsulfonyl) - 2 - chloropropanenitrile.

Preparation of 3-(methylsulfonyl)-2-chloropropanenitrile

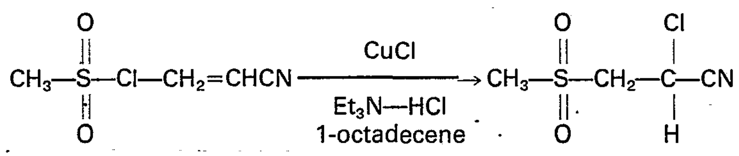


11.5 g (100 m mole of $CH_3SO_2Cl$, 26 ml (21 g, 400 m mole) of acrylonitrile, 200 mg (2 m mole) of CuCl, 400 mg (3 m mole) of $Et_3N \cdot HCl$ and 10 drops of octadecene were mixed and heated to reflux in an atmosphere of nitrogen at about atmospheric pressure $N_2$. A temperature of 85°C was maintained. The reaction was continued, under reflux conditions for about 18 hours, after which the reaction mixture was found to be perfectly clear and amber in color. Refluxing was continued for a total time of 26 hours; after which the mixture was allowed to stand overnight. The mixture solidified and was dissolved in $CH_2Cl_2$/ethyl acetate, washed with water, dried and concentrated to a pale yellow oil by vacuum distillation of the solvents. The yield of 3 - (methylsulfonyl) - 2 - chloropropanenitrile was found to be 17.0 g. The product solidified, upon standing, and was found to have a melting point of 64—68°C. It was submitted for I.R. and NMR analysis and was found to be 95% pure 3 - (methylsulfonyl) - 2 - chloropropanenitrile.

**Claims**

1. An antimicrobial composition characterised in that it comprises an antimicrobial effective amount of 3 - (methylsulfonyl) - 2 - chloropropanenitrile, optionally with an adjuvant.

2. A herbicidal composition characterised in that it comprises a herbicidally effective amount of 3 - (methylsulfonyl) - 2 - chloropropanenitrile optionally with an adjuvant.

**Patentansprüche**

1. Mikrobizide Mischung, dadurch gekennzeichnet, daß sie 3 - Methylsulfonyl - 2 - chlorpropannitril in mikrobizidwirksamer Menge und gegebenenfalls einen Hilfsstoff enthält.

2. Herbizide Mischung, dadurch gekennzeichnet, daß sie 3 - Methylsulfonyl - 2 - chlorpropannitril in herbizidwirksamer Menge und gegebenenfalls einen Hilfsstoff enthält.

**Revendications**

1. Une composition antimicrobienne, caractérisé en ce qu'elle comprend une quantité efficace quant au caractère antimicrobien de 3 - (méthylsulfonyl) - 2 - chloropropanenitrile, et un éventuel adjuvant.

2. Une composition herbicide, caractérisé en ce qu'elle comprend une quantité efficace quant au caractère herbicide de 3 - (méthylsulfonyl) - 2 - chloropropanenitrile et un éventuel adjuvant.